# EUROPÄISCHE PATENTSCHRIFT

(11) **EP 2 628 468 B1**
(45) Veröffentlichungstag und Bekanntmachung des Hinweises auf die Patenterteilung: **30.05.2018**
(21) Anmeldenummer: 13155333.1
(22) Anmeldetag: 15.02.2013
(51) Int. Cl.: A61F 2/91, A61F 2/88

(54) **Medizinische Vorrichtung**
Medical device
Dispositif médical

(30) Priorität: 17.02.2012 DE 102012101294
(43) Veröffentlichungstag der Anmeldung: 21.08.2013
(73) Patentinhaber: Acandis GmbH & Co. KG, 76327 Pfinztal (DE)
(72) Erfinder: Cattaneo, Giorgio, 76199 Karlsruhe (DE); Nagl, Frank, 76137 Karlsruhe (DE); Mailänder, Werner, 75331 Engelsbrand Grunbach (DE)
(74) Vertreter: Kilchert, Jochen

(56) Entgegenhaltungen:
- DE-T2- 69 934 244
- US-A- 5 514 154
- US-A1- 2004 133 271
- US-A1- 2010 004 735

## Beschreibung

Die Erfindung betrifft eine medizinische Vorrichtung mit einer radial komprimierbaren und expandierbaren Gitterstruktur. Derartige Vorrichtungen sind aus der Praxis, beispielsweise in Form von Stents oder Thrombenfängern, bekannt.

Bekannte Stents weisen eine Gitterstruktur auf, die aus Zellen gebildet ist. Die Zellen sind durch miteinander verbundene Stege begrenzt. Insbesondere sind Stents bekannt, die aus Rohren geschnitten sind, beispielsweise durch Laserschneiden. Ein lasergeschnittener Stent ist beispielweise aus DE 699 34 244 T2 bekannt. Der darin beschriebene Stent weist helixförmig verlaufende Stegreihen mit mäanderförmig angeordneten Stegen auf, wobei die Stegreihen durch Verbindungsstege verbunden sind, die eine kleinere Stegbreite als die mäanderförmig angeordneten Stege aufweisen.

Stents sind im Allgemeinen radial komprimierbar und expandierbar. Zur Einführung in ein Blutgefäß wird der Stent im komprimierten Zustand über einen Katheter eingeführt. Nach Verlassen des Katheters weitet sich der Stent aus bzw. expandiert und schmiegt sich an die Gefäßwand an. Dabei versucht der Stent eine kreiszylinderförmige Form einzunehmen. Mit anderen Worten expandiert der Stent radial gleichmäßig.

Die radial gleichmäßige Expansion des Stents ist in geraden Blutgefäßen vergleichsweise unproblematisch. Es hat sich jedoch gezeigt, dass beim Einsetzen des Stents in gekrümmte Blutgefäße Kräfte auftreten, die eine gleichmäßig radiale Expansion des Stents verhindern können. Insbesondere weitet sich der Stent in Querrichtung, also parallel zur Krümmungsachse des Blutgefäßes, stärker aus, als in einer Richtung senkrecht zur Krümmungsachse des Blutgefäßes. Mit anderen Worten stellt sich in Gefäßkrümmungen ein im Wesentlichen ovales Querschnittsprofil des Stents ein. Es besteht daher das Risiko, dass der Stent abschnittsweise unterschiedliche Radialkräfte auf die Gefäßwand ausübt. Dies kann zu einer zusätzlichen lokalen Reizung der Gefäßwände führen. Ferner besteht das Risiko, dass zwischen der Gitterstruktur des Stents und dem Blutgefäß abschnittsweise Abstände bestehen. Da durch die Gitterstruktur die Blutströmung im Bereich zwischen der Gitterstruktur und der Blutgefäßwand beeinflusst wird, können sich darin Blutgerinnsel bilden.

Die Aufgabe der Erfindung besteht darin, eine medizinische Vorrichtung anzugeben, die eine gute Anpassung an eine Gefäßwand ermöglicht, insbesondere in gekrümmten Körperhohlorganen.

Erfindungsgemäß wird diese Aufgabe durch den Gegenstand des Patentanspruchs 1 gelöst.

Demnach beruht die Erfindung auf dem Gedanken, eine medizinische Vorrichtung mit einer radial komprimierbaren und expandierbaren Gitterstruktur anzugeben, die wenigstens eine geschlossene Zelle aufweist. Die geschlossene Zelle ist durch jeweils vier einstückig miteinander gekoppelte Stege begrenzt. Zwei dieser Stege sind als Stabilisierungsstege mit einer ersten Stegbreite b₁ ausgebildet. Zwei weitere Stege sind als Verbindungsstege mit einer zweiten Stegbreite b₂ ausgebildet. Die Verbindungsstege sind innerhalb der Zelle parallel gegenüberliegend angeordnet und durch die Stabilisierungsstege miteinander verbunden. Das Verhältnis b₁/b₂ zwischen der ersten Stegbreite b₁ und der zweiten Stegbreite b₂ beträgt mindestens 1,2.

Erfindungsgemäß ist vorgesehen, dass die Verbindungsstege im Wesentlichen S-förmig ausgebildet sind. Durch die S-förmige Ausbildung der Verbindungsstege wird erreicht, dass die Verbindungsstege leichter verformbar sind. Damit wird die Flexibilität, insbesondere Biegeflexibilität, der Gitterstruktur weiter verbessert. Auch bei einer im Wesentlichen ungeraden Form der Verbindungsstege verlaufen diese innerhalb einer Zelle parallel zueinander. Mit anderen Worten weisen die Verbindungsstege, vorzugsweise alle Verbindungsstege der Gitterstruktur, denselben Verlauf auf, so dass sich insgesamt eine parallele Anordnung ergibt. Zwischen zwei in Umfangsrichtung der Gitterstruktur benachbarten Verbindungsstegen besteht also ein gleichmäßiger Abstand.

Die Erfindung geht auf die Idee zurück, zwei Stege einer Zelle der Gitterstruktur breiter auszubilden als die beiden anderen Stege. Dabei kann die Gitterstruktur eine einheitliche Wandstärke aufweisen. Mit anderen Worten weisen alle Stege der Zelle dieselbe Stegdicke auf, wobei die Stegdicke in radialer Richtung bezogen auf die Längsachse der Gitterstruktur zu messen ist.

Die Erfindung bezieht sich explizit auf eine medizinische Vorrichtung mit einer Gitterstruktur aus geschlossenen Zellen (closed cell design). Das bedeutet, dass jeder Steg einer Zelle an jedem seiner Längsenden mit wenigstens zwei weiteren Stegen, insbesondere wenigstens einem Steg derselben Zelle und einem Steg einer angrenzenden Zelle, verbunden ist. Bei einer Gitterstruktur mit geschlossenen Zellen sind alle Stege mit Verbindern gekoppelt, die die Stege mehrerer Zellen miteinander verbinden. Im Gegensatz dazu weisen Gitterstrukturen mit offenen Zellen (open cell design) Stege auf, die innerhalb einer Zelle miteinander verbunden sind und einen in der Zelle frei angeordneten Endverbinder aufweisen. Die Gitterstruktur bei der erfindungsgemäßen medizinischen Vorrichtung ist vorzugsweise endverbinderfrei bzw. weist wenigstens eine endverbinderfreie Zelle, also eine geschlossene Zelle, auf.

Bei der erfindungsgemäßen medizinischen Vorrichtung ist grundsätzlich nicht ausgeschlossen, dass beispielsweise an den axialen Enden der Gitterstruktur Halteelemente innerhalb einer geschlossenen Zelle angeordnet sind, die zwei miteinander verbundene Stege aufweisen, wobei der Verbindungspunkt zwischen den beiden Stegen frei in der Zelle angeordnet ist. Die Halteelemente können den Zweck haben, sich bei der Platzierung in einem Blutgefäß, insbesondere in einem gekrümmten Blutgefäß, aus der Wandungsebene der Gitterstruktur herauszuheben. Dadurch kann die Gitterstruktur gut in einem Blutgefäß verankert werden. Insofern ist es bei der erfindungsgemäßen medizinischen Vorrichtung durchaus möglich, dass die Gitterstruktur unterschiedliche Zellen aufweist, insbesondere teils durch geschlossene Zellen und teils durch geschlossene Zellen mit Halteelementen gebildet ist. Im Allgemeinen ist es vorteilhaft, wenn die gesamte Gitterstruktur einstückig bzw. einteilig ausgebildet ist. Dies kann beispielsweise durch Herstellen der Gitterstruktur aus einem Vollrohr gewährleistet werden. Insbesondere kann die Gitterstruktur durch Laserschneiden hergestellt sein.

Die Bildung der Gitterstruktur durch weitgehend geschlossene Zellen - das schließt geschlossene Zellen mit Halteelementen ein - hat den Vorteil, dass die Gitterstruktur nach einer teilweisen Entlassung aus einem Katheter bzw. allgemeinen einem Zuführsystem wieder in den Katheter bzw. das Zuführsystem zurückziehbar ist. Da die Zellen geschlossen sind, können sich keine vorstehenden, insbesondere freien, Endverbinder beim Zurückziehen verhaken. Bei geschlossenen Zellen, die Halteelemente aufweisen, sind die Halteelemente vorzugsweise derart ausgerichtet, dass die durch die beiden miteinander verbundenen Stege der Halteelemente gebildete Spitze bezogen auf das Zuführsystem in distale Richtung weist. Dadurch wird verhindert, dass sich das Halteelement beim Zurückziehen der Gitterstruktur in einen Katheter über die Katheterwandung schiebt und ein weiteres Zurückziehen blockiert.

Bei der Erfindung wird durch die unterschiedlichen Stegbreiten der Stege innerhalb der geschlossenen Zelle insgesamt die Flexibilität der Gitterstruktur erhöht. Dies ist besonders anschaulich bei der Expansion der Gitterstruktur in einem gekrümmten Körperhohlorgan erkennbar. Bei der Anordnung der Gitterstruktur in einem gekrümmten Körperhohlorgan wird der Wandungsbereich, der dem Krümmungsmittelpunkt des Körperhohlorgans näher liegt als ein gegenüberliegender Wandungsabschnitt relativ stärker gekrümmt. Dies führt zu einer axialen Stauchung der Zellen im stärker gekrümmten Wandungsbereich der Gitterstruktur. Um einen guten Kontakt zwischen der Gitterstruktur und der Gefäßwand sicherzustellen, ist die Biegeflexibilität der Gitterstruktur entscheidend. Bei der Erfindung wird die Biegeflexibilität durch die unterschiedlichen Stegbreiten zwischen den Stabilisierungsstegen und den Verbindungsstegen erhöht. Dazu ist vorzugsweise vorgesehen, dass die Gitterstruktur zumindest in dem Wandungsbereich, der bei Anordnung der Gitterstruktur in einem gekrümmten Körperhohlorgan stärker gebogen wird als andere Wandungsbereiche, Zellen aufweist, die aus Stabilisierungsstegen und Verbindungsstegen gebildet sind. Die relativ schmaleren Verbindungsstege verformen sich bei der Stauchung bzw. axialen Kompression der Gitterstruktur bzw. eines Wandungsbereichs der Gitterstruktur stärker als die relativ breiteren Stabilisierungsstege. Zusätzlich können die relativ schmaleren Verbindungsstege gegenüber den relativ breiteren Stabilisierungsstegen stärker tordieren, also in sich verdrehen. Das führt dazu, dass die Gitterstruktur insgesamt eine höhere Flexibilität aufweist als Gitterstrukturen mit geschlossenen Zellen, deren Stege eine einheitliche Stegbreite aufweisen.

Über die unterschiedlichen Stegbreiten zwischen den Stabilisierungsstegen und den Verbindungsstegen kann einerseits die Radialkraft der Gitterstruktur eingestellt werden und andererseits eine erhöhte Flexibilität gewährleistet werden. Die Stabilisierungsstege verleihen der Gitterstruktur die nötige Stabilität und Radialkraft, um ein Blutgefäß zu stützen. Die Verbindungsstege weisen hingegen eine kleinere Stegbreite auf, um eine erhöhte Flexibilität, insbesondere Biegeflexibilität, der Gitterstruktur sicherzustellen. Das erfindungsgemäße Verhältnis zwischen der ersten Stegbreite der Stabilisierungsstege und der zweiten Stegbreite der Verbindungsstege von wenigstens 1,2 hat sich als besonders geeignet herausgestellt, um einerseits eine ausreichende Gefäßstützung zu gewährleisten und andererseits eine hohe Flexibilität, insbesondere Biegeflexibilität, zu bieten.

Im Allgemeinen kann vorgesehen sein, dass die Gitterstruktur in einem Ruhezustand, also in einem vollständig expandierten Zustand, ohne Einfluss äußerer Kräfte, eine kreiszylinderförmige bzw. rohrförmige Gestalt einnimmt.

Bei einer bevorzugten Ausführungsform der medizinischen Vorrichtung ist vorgesehen, dass das Verhältnis zwischen der ersten Stegbreite b₁ und der zweiten Stegbreite b₂ mindestens 1,5, insbesondere mindestens 1,8, insbesondere mindestens 2, insbesondere mindestens 2,5, insbesondere mindestens 3, insbesondere mindestens 4, insbesondere mindestens 5, insbesondere mindestens 6, insbesondere mindestens 8, insbesondere mindestens 10, insbesondere mindestens 12, beträgt.

Um eine symmetrische Zelle zu bilden, sind in bevorzugter Weise wenigstens zwei Stabilisierungsstege vorgesehen. Von den vier Stegen einer Zelle können also zwei Stege als Stabilisierungsstege mit der ersten Stegbreite b₁ ausgebildet sein. Die beiden anderen Stege bilden die Verbindungsstege mit der zweiten Stegbreite b₂. Die Zelle ist vorzugsweise abwechselnd durch Stabilisierungsstege und Verbindungsstege begrenzt, d.h. zwischen die Verbindungsstege verbinden die Stabilisierungsstege. Die Stabilisierungsstege und die Verbindungsstege können jeweils parallel gegenüberliegend angeordnet sein.

Ferner kann vorgesehen sein, dass die Gitterstruktur mehrere benachbarte geschlossene Zellen aufweist, deren Stabilisierungsstege miteinander fluchtend verbunden sind, so dass wenigstens eine Stabilisierungsstegreihe gebildet ist, die sich helixförmig um eine Längsachse der Gitterstruktur windet. Mit anderen Worten kann die Stabilisierungsstegreihe die Form einer Schraubenfeder, insbesondere einer gewundenen Torsionsfeder, einnehmen. Die Stabilisierungsstegreihe ist dabei durch zueinander fluchtend angeordnete Stabilisierungsstege gebildet, die direkt miteinander verbunden sind. Auf diese Weise wird eine besonders hohe Biegeflexibilität der Gitterstruktur erreicht, insbesondere über die gesamte Länge der Gitterstruktur.

Vorzugsweise sind wenigstens zwei Stabilisierungsstegreihen gebildet, die durch die Verbindungsstege miteinander verbunden sind. Die Verbindungsstege können in regelmäßigen Abständen zwischen zwei Stabilisierungsstegreihen angeordnet sein, um ein einheitliches, flexibles Verhalten der Gitterstruktur sicherzustellen.

Zu einer gleichmäßigen Flexibilität in zumindest einem Abschnitt der Gitterstruktur trägt es bei, wenn die Verbindungsstege benachbarter geschlossener Zellen zueinander parallel angeordnet sind. Die Verbindungsstege benachbarter geschlossener Zellen können also bezogen auf die Längsachse der Gitterstruktur denselben Winkel bzw. dieselbe Orientierung aufweisen.

Bei einer weiteren bevorzugten Ausführungsform ist vorgesehen, dass die Stabilisierungsstege im Wesentlichen einen geradlinigen Verlauf aufweisen. Auf diese Weise nähert sich die Form einer Stabilisierungsstegreihe weiter an die Form einer Schraubenfeder an. Dies trägt zur verbesserten Crimpbarkeit und Biegeflexibilität der Gitterstruktur bei.

Vorzugsweise weisen die Stabilisierungsstege eine erste Steglänge und die Verbindungsstege eine zweite Steglänge auf, wobei die erste Steglänge und die zweite Steglänge unterschiedlich sind. Auf diese Weise kann die Flexibilität und/oder die Biegbarkeit der Gitterstruktur für unterschiedliche Anwendungsfälle eingestellt werden.

So ist in einer bevorzugten Ausführungsform vorgesehen, dass das Verhältnis der ersten Steglänge zur zweiten Steglänge mindestens 1,2, insbesondere mindestens 1,5, insbesondere mindestens 2, insbesondere mindestens 2,5, insbesondere mindestens 3, insbesondere mindestens 4, insbesondere mindestens 5, beträgt. Bei dieser Ausführungsform, bei der die Stabilisierungsstege, die die erste Steglänge aufweisen, länger als die Verbindungsstege sind, die die zweite Steglänge aufweisen, wird vorteilhaft erreicht, dass die Flexibilität, insbesondere Biegeflexibilität, und die Crimpbarkeit der Gitterstruktur beim Einsatz der medizinischen Vorrichtung in Körperhohlorganen, insbesondere Blutgefäßen, mit relativ kleinen Durchmessern erhöht werden.

Alternativ kann vorgesehen sein, dass das Verhältnis der zweiten Steglänge zur ersten Steglänge mindestens 1,2, insbesondere mindestens 1,5, insbesondere mindestens 2, insbesondere mindestens 2,5, insbesondere mindestens 3, insbesondere mindestens 4, insbesondere mindestens 5, beträgt. In dieser Ausführungsform weisen also die Verbindungsstege eine größere Steglänge als die Stabilisierungsstege auf. Dadurch wird die Feinmaschigkeit der Gitterstruktur erhöht bzw. die Zellengröße reduziert. Da die Verbindungsstege eine relativ hohe Verformbarkeit aufweisen, wird die Flexibilität, insbesondere Biegeflexibilität, der Gitterstruktur kaum beeinträchtigt. In dieser Ausführungsform eignet sich die medizinische Vorrichtung insbesondere zur Beeinflussung von Strömungsverhältnissen in einem Aneurysma, also zur Platzierung in einem Blutgefäßabschnitt, aus dem ein Aneurysma hervorgeht. Alternativ kann die medizinische Vorrichtung vorteilhaft bei der Behandlung von Stenosen eingesetzt werden, wobei die erhöhte Feinmaschigkeit verhindert, dass Plaqueteile der Stenose in die Blutströmung gelangen.

Bei einer bevorzugten Ausführungsform der erfindungsgemäßen medizinischen Vorrichtung weist die Gitterstruktur höchstens 8, insbesondere höchstens 6, insbesondere höchstens 4, insbesondere höchstens 3, insbesondere höchstens 2, Stabilisierungsstegreihen auf. Die Stabilisierungsstegreihen können in regelmäßigen Abständen um den Umfang der Gitterstruktur angeordnet sein. Insbesondere kann vorgesehen sein, dass zwischen jeweils zwei Stabilisierungsstegreihen eine Stegreihe helixförmig um die Längsachse der Gitterstruktur verläuft, die durch Stege gebildet ist, die eine andere, insbesondere kleinere, Stegbreite als die Stabilisierungsstege aufweisen.

Konkret ist in einer bevorzugten Ausführungsform vorgesehen, dass die Gitterstruktur mehrere Stegreihen aufweist, die parallel zueinander verlaufen und sich in der gleichen Windungsrichtung um die Längsachse der Gitterstruktur winden. Die Stegreihen sind durch zueinander fluchtende und miteinander verbundene Stege einzelner Zellen gebildet, wobei die Stege einen geradlinigen Verlauf aufweisen. Wenigstens zwei der helixförmig um die Längsachse der Gitterstruktur gewundenen Stegreihen sind durch aneinandergereihte bzw. miteinander fluchtend verbundene Stabilisierungsstege gebildet. Wenigstens zwei der Stegreihen bilden also Stabilisierungsstegreihen. Es können mehrere Stabilisierungsstegreihen vorgesehen sein, wobei in besonders bevorzugter Ausgestaltung der Erfindung die Stabilisierungsstegreihen in regelmäßigen Abständen um den Umfang der Gitterstruktur verteilt angeordnet sind. Insbesondere kann die Gitterstruktur musterartig ausgebildet sein, so dass jeweils in Umfangsrichtung der Gitterstruktur eine sich wiederholende Abfolge aus Stegreihen mit und ohne Stabilisierungsstege vorgesehen ist.

Überdies kann bei der erfindungsgemäßen medizinischen Vorrichtung vorgesehen sein, dass die Gitterstruktur in Umfangsrichtung wenigstens 6, insbesondere wenigstens 8, insbesondere wenigstens 12, insbesondere wenigstens 18, insbesondere wenigstens 24, insbesondere wenigstens 32, insbesondere wenigstens 36, insbesondere wenigstens 48, insbesondere wenigstens 56, insbesondere wenigstens 64, Verbindungsstege aufweist. Die vorgenannte Anzahl an Verbindungsstegen wird in Umfangsrichtung ermittelt, d.h. entscheidend ist die Anzahl der Verbindungsstege, die eine einzelne Querschnittsebene der Gitterstruktur schneiden. Über die Anzahl der Verbindungsstege kann einerseits die Biegeflexibilität der Gitterstruktur bzw. allgemein der medizinischen Vorrichtung beeinflusst werden. Andererseits wird durch eine Erhöhung der Anzahl der Verbindungsstege gleichzeitig die Feinmaschigkeit der Gitterstruktur erhöht. Eine hohe Anzahl an Verbindungsstegen, also eine erhöhte Feinmaschigkeit, ist für medizinische Vorrichtungen zweckmäßig, die beispielsweise zur Abdeckung eines Aneurysmas genutzt werden.

Die Anzahl der Verbindungsstege der Gitterstruktur kann begrenzt sein. Insbesondere kann vorgesehen sein, dass die Gitterstruktur in Umfangsrichtung höchstens 120, insbesondere höchstens 100, insbesondere höchstens 80, insbesondere höchstens 70, insbesondere höchstens 60, insbesondere höchstens 50, Verbindungsstege aufweist. Die vorgenannten Obergrenzen stellen einerseits eine gute Strömungsbeeinflussung aufgrund der hohen Feinmaschigkeit, beispielsweise zur Behandlung eines Aneurysmas, sicher und stellen andererseits eine noch ausreichende Biegeflexibilität sicher, so dass die Gitterstruktur gut in einem gekrümmten Körperhohlorgan platziert werden kann.

Zur Gewährleistung der für die Erfindung zweckmäßigen Biegestabilität der Gitterstruktur ist es ferner bevorzugt, wenn die zweite Stegbreite b₂ höchstens 35 µm, insbesondere höchstens 30 µm, insbesondere höchstens 25 µm, insbesondere höchstens 20 µm, insbesondere höchstens 15 µm, beträgt.

Überdies hat es sich als förderlich für die Biegeflexibilität im Allgemeinen und die Crimpbarkeit innerhalb eines Zuführsystems bzw. Katheters in Speziellen herausgestellt, wenn die Stabilisierungsstege und die Verbindungsstege bezogen auf die Längsachse der Gitterstruktur denselben Winkel einnehmen, jeweils mit umgekehrten Vorzeichen. Das bedeutet, dass die Winkelhalbierende zwischen einem Verbindungssteg und einem in Umfangsrichtung benachbart angeordneten und mit dem Verbindungssteg direkt verbundenen Stabilisierungssteg parallel zur Längsachse verläuft. Der Winkel bei gekrümmten, insbesondere S-förmigen, Stegen wird zwischen der Längsachse bzw. einer Projektion der Längsachse in der Wandungsebene der Gitterstruktur und einer virtuellen Verbindungsgeraden ermittelt, die die beiden Enden des gekrümmten Stegs verbindet.

Die Erfindung wird im Folgenden anhand von Ausführungsbeispielen unter Bezugnahme auf die beigefügten, schematischen Zeichnungen näher erläutert. Darin zeigen:
- Fig. 1a:: eine Draufsicht auf eine Zelle der Gitterstruktur einer erfindungsgemäßen medizinischen Vorrichtung nach einem bevorzugten Ausführungsbeispiel im Ruhezustand;
- Fig. 1b:: die Zelle gemäß Fig. 1a unter Einwirkung von axialen Kompressionskräften;
- Fig. 2a:: eine Seitenansicht der Gitterstruktur einer erfindungsgemäßen medizinischen Vorrichtung nach einem weiteren bevorzugten Ausführungsbeispiel im implantierten Zustand in einem Körperhohlorgan mit einem relativ großen Querschnittsdurchmesser;
- Fig. 2b:: die Gitterstruktur gemäß Fig. 2a im implantierten Zustand in einem Körperhohlraum mit relativ kleinem Querschnittsdurchmesser;
- Fig. 3:: eine Seitenansicht der Gitterstruktur einer erfindungsgemäßen medizinischen Vorrichtung nach einem weiteren bevorzugten Ausführungsbeispiel im implantierten Zustand, wobei die Verbindungsstege eine größere Steglänge als die Stabilisierungsstege aufweisen; und
- Fig. 4:: eine Seitenansicht der Gitterstruktur einer erfindungsgemäßen medizinischen Vorrichtung nach einem weiteren bevorzugten Ausführungsbeispiel im implantierten Zustand, wobei die Stabilisierungsstege eine größere Steglänge als die Verbindungsstege aufweisen.

Es hat sich gezeigt, dass bei der Implantation einer expandierbaren Gitterstruktur 10 in einem Körperhohlorgan, insbesondere einem Blutgefäß 30, das eine Gefäßkrümmung aufweist, die einzelnen Zellen 15 der Gitterstruktur 10 unterschiedlich verformt werden, um dem Verlauf des Blutgefäßes 30 zu folgen. Während die bezogen auf den Krümmungsmittelpunkt des Blutgefäßes 30 bzw. des gekrümmten Blutgefäßabschnitts radial äußeren Bereiche der Gitterstruktur 10 gestreckt werden, werden die radial inneren Wandungsabschnitte im Wesentlichen gestaucht. Für die einzelnen Zellen 15 der Gitterstruktur 10 bedeutet dies, dass Zellen, die vom Krümmungsmittelpunkt der Blutgefäßkrümmung weiter beabstandet angeordnet sind, eine größere axiale Ausdehnung aufweisen als Zellen 15, die in einem Wandungsabschnitt der Gitterstruktur 10 angeordnet sind, der dem Krümmungsmittelpunkt der Blutgefäßkrümmung näher liegt.

Die Zelle 15 der Gitterstruktur 10 weist im Wesentlichen vier Stege 11, 12, 13, 14 auf, die die Zelle 15 bzw. die Zellenfläche begrenzen. Die vier Stege 11, 12, 13, 14 sind miteinander verbunden, wobei zwischen jeweils zwei Stegen 11, 12, 13, 14 ein Verbinder 21, 22, 23, 24 angeordnet ist. Die Verbinder 21, 22, 23, 24 bilden im Wesentlichen Knotenpunkte oder Verknüpfungsstellen zwischen zwei Stegen 11, 12, 13, 14 einer einzigen Zelle 15. Gleichzeitig können die Verbinder 21, 22, 23, 24 Knotenpunkte bzw. Verknüpfungsstellen zu angrenzenden Zellen 15 bilden, also insgesamt mehr als zwei Stege 11, 12, 13, 14 miteinander koppeln, wobei wenigstens einer der gekoppelten Stege 11, 12, 13, 14 einer benachbarten Zelle 15 angehört.

Der einzelnen Zelle 15 sind insgesamt vier Verbinder 21, 22, 23, 24 zugeordnet. Dabei verbindet ein erster Verbinder 21 einen ersten Stabilisierungssteg 11 mit einem ersten Verbindungssteg 12. Ein zweiter Verbinder 22 koppelt den ersten Verbindungssteg 12 mit einem zweiten Stabilisierungssteg 13. Der zweite Stabilisierungssteg 13 ist durch einen dritten Verbinder 23 mit dem zweiten Verbindungssteg 14 gekoppelt. Ein vierter Verbinder 24 verbindet den zweiten Verbindungssteg 14 mit dem ersten Stabilisierungssteg 11.

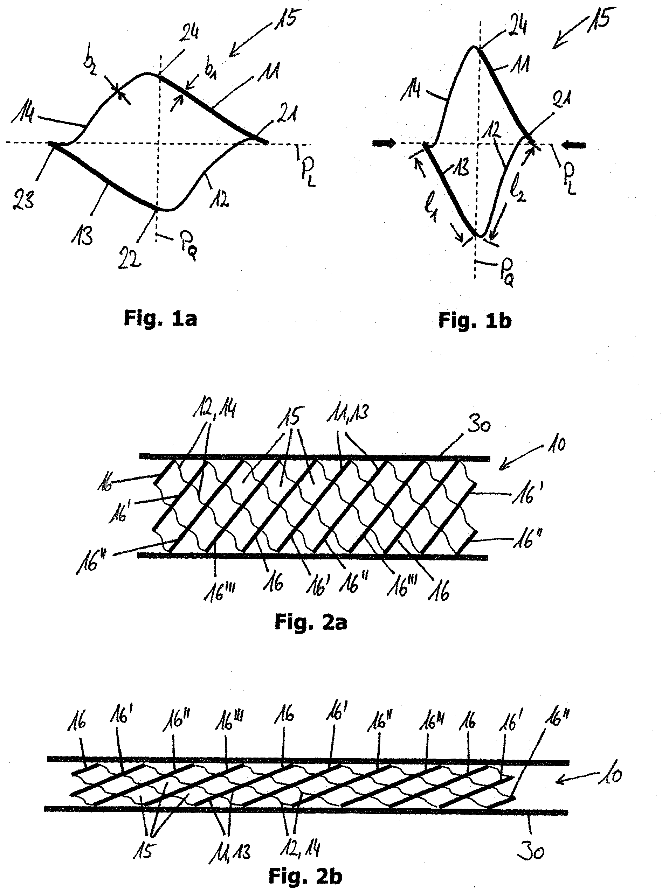

Fig. 1a zeigt eine Zelle 15 einer Gitterstruktur 10 der erfindungsgemäßen medizinischen Vorrichtung nach einem bevorzugten Ausführungsbeispiel. Die medizinische Vorrichtung kann generell einen Stent, einen Thrombenfänger und/oder einen Blutfilter umfassen. In bevorzugter Weise ist die Gitterstruktur 10 zumindest abschnittsweise kreiszylindrisch ausgebildet. Generell ist die Gitterstruktur 10 radial komprimierbar und expandierbar. Die Gitterstruktur 10 kann also einen radial komprimierten, d.h. gecrimpten, Zustand aufweisen, der zur Zufuhr der Gitterstruktur 10 in ein Körperhohlorgan, insbesondere ein Blutgefäß 30, geeignet ist. Ferner kann die Gitterstruktur 10 einen radial expandierten Zustand aufweisen, in welchem die Gitterstruktur 10 einen größeren Querschnittsdurchmesser aufweist als im radial komprimierten Zustand. Der radial expandierte Zustand der Gitterstruktur 10 umfasst auch einen implantierten Zustand der Gitterstruktur 10, wobei die Gitterstruktur 10 eine Radialkraft auf das Körperhohlorgan, insbesondere das Blutgefäß 30, ausübt. Ein weiterer expandierter Zustand der Gitterstruktur 10 betrifft den Ruhezustand bzw. Herstellzustand, in dem die Gitterstruktur 10 frei von äußeren Kräften ist.

Von den vier Stegen 11, 12, 13, 14 der Zelle 15 sind jeweils zwei gegenüberliegend angeordnete Stege 11, 13, 12, 14 gleichartig ausgebildet. Konkret ist vorgesehen, dass die Zelle 15 zwei Stabilisierungsstege 11, 13 aufweist, die einander gegenüberliegend angeordnet sind. Die Stabilisierungsstege 11, 13 sind insbesondere durch zwei Verbindungsstege 12, 14 voneinander getrennt, wobei die Verbindungsstege 13, 14 ebenfalls gleichartig ausgebildet sind. Die Stabilisierungsstege 11, 13 und die Verbindungsstege 12, 14 unterscheiden sich voneinander. Insbesondere ist vorgesehen, dass die Stabilisierungsstege 11, 13 eine erste Stegbreite b₁ und die Verbindungsstege 12, 14 eine zweite Stegbreite b₂ aufweisen. Die erste Stegbreite b₁ der Stabilisierungsstege 11, 13 ist größer als die zweite Stegbreite b₂ der Verbindungsstege 12, 14. Insbesondere hat es sich als vorteilhaft herausgestellt, wenn die erste Stegbreite b₁ in einem Verhältnis zur zweiten Stegbreite b₂ (b₁/b₂) steht, das wenigstens 1,2, insbesondere wenigstens 1,5, insbesondere wenigstens 1,8, insbesondere wenigstens 2, insbesondere wenigstens 2,5, insbesondere wenigstens 3, insbesondere wenigstens 4, insbesondere wenigstens 5, insbesondere wenigstens 6, beträgt.

Die Stabilisierungsstege 11, 13 bilden im Wesentlichen eine Stützstruktur der Gitterstruktur 10. Hingegen sorgen die Verbindungsstege 12, 14 für eine erhöhte Flexibilität der Gitterstruktur 10. Dazu kann, wie in Fig. 1a dargestellt ist, vorteilhaft vorgesehen sein, dass die Verbindungsstege 12, 14 eine S-förmige Gestalt aufweisen, sich also S-förmig zwischen den Stabilisierungsstegen 11, 13 erstrecken. Die Zelle 15 gemäß Fig. 1a ist hinsichtlich der axialen Kräfte und der Umfangskräfte im Kräftegleichgewicht. Das bedeutet, dass Kräfte, die entlang der Längsachse der Gitterstruktur 10 wirken und Kräfte, die entlang der Längsrichtung der Gitterstruktur 10 wirken, im Wesentlichen denselben Betrag aufweisen. In Fig. 1 ist durch gestrichelte Linien einerseits die Projektion P_{L} der Längsachse der Gitterstruktur auf die Wandungsebene der Gitterstruktur und andererseits die Projektion P_{Q} der Querachse der Gitterstruktur 10 gezeigt. Die Querachse entspricht einer Achse der Gitterstruktur, die senkrecht zur Längsachse angeordnet ist und sich in einer Querschnittsebene der Gitterstruktur 10 erstreckt.

In Fig. 1b ist die Zelle 15 gemäß Fig. 1a dargestellt, wenn axiale Kräfte auf die Zelle 15 wirken. Dies kann der Fall sein, wenn die Zelle 15 in einem Bereich der Umfangswandung der Gitterstruktur 10 angeordnet ist, der im implantierten Zustand innerhalb einer Gefäßkrümmung näher am Krümmungsmittelpunkt zu liegen kommt. Es ist gut erkennbar, dass sich die Zelle 15 stark verformt, insbesondere in Umfangsrichtung, also entlang der Projektion P_{Q} der Querachse, gestreckt wird. Konkret wird die Zelle 15 in axialer Richtung gestaucht, wie durch die Pfeile in Fig. 1b angedeutet ist, so dass der zweite Verbinder 22 und der vierte Verbinder 24 in Umfangsrichtung der Gitterstruktur 10 auseinander gedrängt werden. Aufgrund der unterschiedlichen Stegbreiten, insbesondere der relativ kleineren Stegbreite b₂ der Verbindungsstege 12, 14, lässt sich die Zelle 15 leicht in axialer Richtung verformen, so dass sich die Gitterstruktur 10 insgesamt gut an eine Gefäßkrümmung anpassen kann.

Die im Folgenden beschriebenen Figuren 2a bis 4 zeigen eine Gitterstruktur 10 der medizinischen Vorrichtung in einer Seitenansicht, wobei Stege 11, 12, 13, 14, die auf der vom Betrachter abgewandten Seite angeordnet sind, aus Gründen der Übersichtlichkeit nicht dargestellt sind.

In Fig. 2a ist ein Ausführungsbeispiel der gesamten Gitterstruktur 10 dargestellt, die in ein Blutgefäß 30 eingesetzt ist. Die Gitterstruktur 10 liegt also im implantierten Zustand vor. Die Gitterstruktur 10 weist mehrere Zellen 15 auf, wobei alle Zellen 15 der Gitterstruktur 10 gleichartig ausgebildet sind. Insbesondere weist jede der Zellen 15 jeweils zwei Stabilisierungsstege 11, 13 und zwei Verbindungsstege 12, 14 auf. Die Stabilisierungsstege 11, 13 benachbarter Zellen sind einerseits miteinander verbunden und fluchten andererseits zueinander, wobei in Fig. 2a gut erkennbar ist, dass die Stabilisierungsstege 11, 13 einen geradlinigen Verlauf aufweisen. Die Stabilisierungsstege 11, 13 erstrecken sich also jeweils geradlinig zwischen zwei Verbindern 21, 22, 23, 24. Daraus ergeben sich parallel zueinander verlaufende Stabilisierungsstegreihen 16, 16', 16", 16"', wobei bei dem Ausführungsbeispiel gemäß Fig. 2a alle in derselben Windungsrichtung um die Längsachse der Gitterstruktur 10 verlaufenden Stege Stabilisierungsstege 11, 13, also Stabilisierungsstegreihen 16, 16', 16", 16'" bilden. Anhand der Hochindizes ist in den Figuren die Anzahl der Stabilisierungsstegreihen 16, 16,', 16", 16'" kenntlich gemacht, um den helixförmigen Verlauf der Stabilisierungsstegreihen 16, 16,', 16", 16'" zu verdeutlichen. So ist bei den Ausführungsbeispielen gemäß den Fig. 2a bis 4 vorgesehen, dass die Gitterstruktur 10 insgesamt vier Stabilisierungsstegreihen 16, 16', 16", 16'" umfasst, die im Wesentlichen parallel zueinander helixförmig um die Längsachse der Gitterstruktur gewunden sind.

Im Allgemeinen erstreckt sich die Stabilisierungsstegreihe 16 helixförmig, insbesondere schraubenfederartig, um die Längsachse der Gitterstruktur 10. Wenn die Zellen 15, deren Stege die Stabilisierungsstegreihe 16 bilden, jeweils zwei Stabilisierungsstege 11, 13 aufweisen, sind zwei parallel zueinander verlaufende Stabilisierungsstegreihen 16 gebildet. Zwischen den parallel zueinander verlaufenden Stabilisierungsstegreihen 16 sind die Verbindungsstege 12, 14 der Zellen 15 angeordnet (Fig. 2a bis 4). Die Verbindungsstege 12, 14 weisen einen S-förmigen Verlauf auf. Die Stabilisierungsstegreihen 16, die als Schraubenfeder angesehen werden können, werden also durch die Verbindungsstege 12, 14 in Form gehalten. Mit anderen Worten verhindern die Verbindungsstege 12, 14, dass die Stabilisierungsstegreihen 16 bzw. Schraubenfedern unkontrolliert expandieren, also sich bei der Expansion ungleichmäßig voneinander entfernen.

Fig. 2b zeigt dieselbe Gitterstruktur 10 im implantierten Zustand in einem Blutgefäß 30, das einen relativ kleineren Querschnittsdurchmesser aufweist. Obwohl das Blutgefäß gemäß Fig. 2b geradlinig ausgebildet ist, also keine Gefäßkrümmung zeigt, ist die hohe Flexibilität der Gitterstruktur 10 gut erkennbar. Die helixförmig gewundenen Stabilisierungsstegreihen 16 weisen zur Gefäßwand des Blutgefäßes 30 einen flacheren Winkel auf, als bei dem Ausführungsbeispiel gemäß Fig. 2a. Daraus geht hervor, dass über die Neigung der Stabilisierungsstegreihen 16 ein relativ hohes Verhältnis zwischen einem vollständig komprimierten Querschnittsdurchmesser und einem vollständig expandiertem Querschnittsdurchmesser der Gitterstruktur 10 erreicht werden kann. Dies gilt auch für einzelne Abschnitte der Gitterstruktur 10, beispielsweise einem Wandungsbereich, der im implantierten Zustand der Gitterstruktur 10 näher am Krümmungsmittelpunkt einer Gefäßkrümmung angeordnet ist als ein radial gegenüberliegender Wandungsbereich.

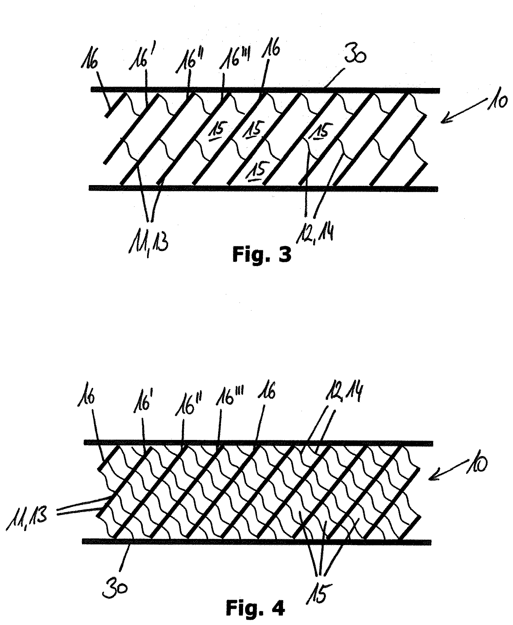

In Fig. 3 ist eine ähnliche Gitterstruktur 10 einer medizinischen Vorrichtung nach einem bevorzugten Ausführungsbeispiel gezeigt, wobei die Stabilisierungsstege 11, 13 der einzelnen Zellen 15 helixförmig angeordnete Schraubenfedern bilden. Die Stabilisierungsstege 11, 13 weisen jeweils einen geradlinigen Verlauf auf. Im Allgemeinen gilt jedoch, dass die Stabilisierungsstege 11, 13 auch eine gekrümmte Form, beispielsweise eine S-Form, aufweisen können.

Bei dem vorhergehenden Ausführungsbeispiel gemäß Figuren 2a und 2b weisen die Stabilisierungsstege 11, 13 und die Verbindungsstege 12, 14 jeweils dieselbe Steglänge auf. Bei dem Ausführungsbeispiel gemäß Figuren 3 und 4 ist hingegen vorgesehen, dass die Stabilisierungsstege 11, 13 eine erste Steglänge l₁ und die Verbindungsstege 12, 14 eine zweite Steglänge l₂ aufweisen, wobei die erste Steglänge l₁ und die zweite Steglänge l₂ unterschiedlich sind.

In Fig. 3 ist eine Gitterstruktur 10 dargestellt, deren Verbindungsstege 12, 14 eine zweite Steglänge l₂ aufweisen, die größer als die erste Steglänge l₁ der Stabilisierungsstege 11, 13 ist. Damit wird eine Gitterstruktur 10 bereitgestellt, die sich durch eine erhöhte Feinmaschigkeit auszeichnet und beispielsweise zur Abdeckung von Aneurysmen geeignet ist. Vorzugsweise besteht zwischen der zweiten Steglänge l₂ und der ersten Steglänge l₁ ein Verhältnis (l₂/l₁) von wenigstens 1,2, insbesondere wenigstens 1,5, insbesondere wenigstens 2, insbesondere wenigstens 2,5, insbesondere wenigstens 3, insbesondere wenigstens 4, insbesondere wenigstens 5.

Ferner ist in Fig. 3 erkennbar, dass die Verbindungsstege 12, 14 in Längsrichtung der Gitterstruktur 10 benachbarter Zellen 15 im Unterschied zu den Ausführungsbeispielen gemäß Fig. 2a, 2b und 4 versetzt zueinander angeordnet sind. Die Verbindungsstege 12, 14 benachbarter Zellen 15 sind miteinander indirekt durch die Stabilisierungsstege 11, 13 gekoppelt. Alternativ können die Verbindungsstege 12, 14 derart angeordnet sein, dass jeweils zwei Verbindungsstege 12, 14 in Längsrichtung benachbarter Zellen 15 fluchtend miteinander verbunden sind, vorzugsweise analog zur fluchtenden Verbindung der Stabilisierungsstege 11, 13. Das Verhältnis l₁/l₂ der Steglängen zwischen den Stabilisierungsstegen 11, 13 und den Verbindungsstegen 12, 14 kann dabei dennoch größer als 1 betragen, insbesondere einen der zuvor genannten Werte annehmen.

Bei dem Ausführungsbeispiel gemäß Fig. 4 ist vorgesehen, dass die Stabilisierungsstege 11, 13 eine erste Steglänge l₁ aufweisen, die größer als die zweite Steglänge l₂ der Verbindungsstege 12, 14 ist. Dadurch wird eine Gitterstruktur 10 bereitgestellt, deren Flexibilität und Crimpbarkeit derart eingestellt ist, dass sich die Gitterstruktur 10 gut zur Implantation in kleine Blutgefäße 30, d.h. in Blutgefäße 30 mit kleinem Querschnittsdurchmesser, eignet. Bei diesem Ausführungsbeispiel ist es besonders vorteilhaft, wenn das Verhältnis (l₁/l₂) der ersten Steglänge l₁ der Stabilisierungsstege 11, 13 und der zweiten Steglänge l₂ der Verbindungsstege 12, 14 wenigstens 1,2, insbesondere wenigstens 1,5, insbesondere wenigstens 2, insbesondere wenigstens 2,5, insbesondere wenigstens 3, insbesondere wenigstens 4, insbesondere wenigstens 5, beträgt.

Die Flexibilität der Gitterstruktur 10 kann zusätzlich dadurch beeinflusst werden, dass der Winkel zwischen zwei in Umfangsrichtung benachbarten Stegen 11, 12, 13, 14 der Zelle 15 entsprechend eingestellt wird. Der Winkel zwischen zwei in Umfangsrichtung benachbarten Stegen 11, 12, 13, 14, beispielsweise dem ersten Stabilisierungssteg 11 und dem ersten Verbindungssteg 12 oder dem zweiten Stabilisierungssteg 13 und dem zweiten Verbindungssteg 14, wird als Tip-Winkel bezeichnet. Vorzugsweise beträgt der Tip-Winkel wenigstens 60°, insbesondere wenigstens 70°, insbesondere wenigstens 80°, insbesondere wenigstens 90°, insbesondere wenigstens 100°, insbesondere wenigstens 110°, insbesondere wenigstens 120°, insbesondere wenigstens 130°, insbesondere wenigstens 140°. Dies gilt für alle Ausführungsbeispiele. Grundsätzlich ist es bevorzugt, wenn die Winkelhalbierende des Tip-Winkels parallel zur Längsachse der Gitterstruktur verläuft, insbesondere der Projektion P_{L} der Längsachse entspricht.

Des Weiteren gilt für alle Ausführungsbeispiele, dass die Wandstärke der Gitterstruktur 10 vorzugsweise gleichmäßig ausgebildet ist. Alle Stege 11, 12, 13, 14 der Gitterstruktur 10, insbesondere alle Stege 11, 12, 13, 14 der Zelle 15, weisen also dieselbe Wandstärke bzw. Stegdicke auf. Diese beträgt vorzugsweise höchstens 90 µm, insbesondere höchstens 80 µm, insbesondere höchstens 70 µm, insbesondere höchstens 60 µm, insbesondere höchstens 50 µm, insbesondere höchstens 40 µm.

Generell kann der Querschnittsdurchmesser der Gitterstruktur 10 im Ruhezustand bzw. Herstellzustand höchstens 12 mm, insbesondere höchstens 8 mm, insbesondere höchstens 6 mm, insbesondere höchstens 5 mm, insbesondere höchstens 4 mm, insbesondere höchstens 3,5 mm, insbesondere höchstens 3 mm, insbesondere höchstens 2,5 mm betragen.

Die erfindungsgemäße medizinische Vorrichtung kann als Stent ausgebildet sein. In diesem Fall weist die Gitterstruktur 10 vorzugsweise zwei axiale, offene Enden auf. Alternativ kann vorgesehen sein, dass die Gitterstruktur 10 mit einem Transportdraht verbunden ist und insbesondere ein geschlossenes axiales Ende aufweist. Dabei kann die erfindungsgemäße medizinische Vorrichtung eine Rekanalisationsvorrichtung, insbesondere ein Thrombenentfernungssystem, konkret einen Thrombenfänger und/oder Blutfilter, bilden.

Im Allgemeinen kann die erfindungsgemäße medizinische Vorrichtung eine Abdeckung aufweisen, insbesondere eine Kunststoffabdeckung. Als bevorzugtes Material für die Abdeckung kann Polyurethan eingesetzt werden. Die Abdeckung überspannt die Gitterstruktur 10, insbesondere die Zellen 15. So kann beispielsweise ein Stent-Graft gebildet werden. Die Abdeckung kann im Wesentlichen fluiddicht ausgebildet sein. Es ist auch möglich, dass die Abdeckung Öffnungen, insbesondere Poren aufweist, so dass die Abdeckung zumindest teilweise fluiddurchlässig ist. Die mit der Abdeckung bespannte Gitterstruktur 10 eignet sich insbesondere zum Einsatz von Blutfiltern und/oder Flow-Divertern.

Im Allgemeinen ist bei der Erfindung vorgesehen, dass die Gitterstruktur wenigstens eine geschlossene Zelle aufweist, die durch jeweils vier einstückig miteinander gekoppelte Stege begrenzt ist, von denen wenigstens ein Steg 11, 12, 13, 14 als Stabilisierungssteg 11, 13 und wenigstens zwei weitere Stege 11, 12, 13, 14 als Verbindungssteg 12, 14 ausgebildet sind. Im Rahmen der Anmeldung wird als geschlossene Zelle 15 eine Zelle bezeichnet, die vollumfänglich in die Gitterstruktur eingebettet ist. Das bedeutet, dass alle Stege 11, 12, 13, 14 der Zelle 15 mit wenigstens einem Steg 11, 12, 13, 14 einer benachbarten Zelle verbunden sind. Die Verbinder 21, 22, 23, 24 der Zelle 15 koppeln also nicht nur zwei Stege 11, 12, 13, 14 einer ersten Zelle 15, sondern verbinden diese Stege 11, 12, 13, 15 auch mit wenigstens einem weiteren Steg 11, 12, 13, 14 einer benachbarten, zweiten Zelle 15. Insbesondere im Hinblick auf die Stabilisierungsstege 11, 13 ist vorgesehen, dass jeder Stabilisierungssteg 11, 13, einer geschlossenen Zelle 15 mit wenigstens einem Stabilisierungssteg 11, 13, einer benachbarten Zelle 15 fluchtend verbunden ist. Die Gitterstruktur 10 kann also wenigstens zwei helixförmig angeordnete Stabilisierungsstegreihen 16 umfassen, die im Wesentlichen parallel zueinander verlaufen. Mit anderen Worten kann die Gitterstruktur 10 durch mehrere parallel verlaufende Schraubenfedern gebildet sein, die durch die Stabilisierungsstege 11, 13 gebildet sind, wobei die einzelnen Schraubenfedern durch die Verbindungsstege 12, 14 miteinander gekoppelt sind. Die Stabilisierungsstege 11, 13 und die Verbindungsstege 12, 14 sind dabei vorzugsweise einstückig bzw. einteilig miteinander verbunden. Die gesamte Gitterstruktur ist einteilig ausgebildet.

Im Hinblick auf die Dimensionen der erfindungsgemäßen medizinischen Vorrichtung, insbesondere der Gitterstruktur 10, ist Folgendes vorgesehen:
Bei einem Durchmesser der Gitterstruktur 10 im Herstellzustand, also im kraftunbelasteten Ruhezustand, zwischen 6 mm und 12 mm beträgt der Innendurchmesser eines Zuführsystems für die Gitterstruktur 10 bzw. die medizinische Vorrichtung vorzugsweise höchstens 2,2 mm, insbesondere höchstens 2 mm, insbesondere höchstens 1,8 mm, insbesondere höchstens 1,6 mm, insbesondere höchstens 1,4 mm, insbesondere höchstens 1,2 mm. In diesem Fall ist vorzugsweise vorgesehen, dass die Gitterstruktur 10 wenigstens 12, insbesondere wenigstens 18, insbesondere wenigstens 24, insbesondere wenigstens 32, insbesondere wenigstens 36, insbesondere wenigstens 48, insbesondere wenigstens 56, insbesondere wenigstens 64, Verbindungsstege 12, 14 aufweist. Die maximale Anzahl von Verbindungsstegen 12, 14 beträgt bei dieser Variante vorzugsweise höchstens 120, insbesondere höchstens 100, insbesondere höchstens 80, insbesondere höchstens 70.

Bei einem Durchmesser der Gitterstruktur 10 im Ruhezustand von 2 mm bis 6 mm kommt vorzugsweise ein Zuführsystem zum Einsatz, dessen Innendurchmesser höchstens 1,0 mm, insbesondere höchstens 0,72 mm, insbesondere höchstens 0,6 mm, insbesondere höchstens 0,51 mm, insbesondere höchstens 0,42 mm beträgt. Die Gitterstruktur 10 kann in diesem Fall wenigstens 12, insbesondere wenigstens 18, insbesondere wenigstens 24, insbesondere wenigstens 32, insbesondere wenigstens 36, insbesondere wenigstens 42, insbesondere wenigstens 48, Verbindungsstege 12, 14 aufweisen. Dabei ist vorgesehen, dass die Gitterstruktur 10 maximal 80, insbesondere maximal 70, insbesondere maximal 60, insbesondere maximal 50, Verbindungsstege 12, 14 umfasst.

Grundsätzlich gilt, dass das Zuführsystem zumindest einen Katheter mit den zuvor genannten Innendurchmesserwerten aufweist.

Für die Herstellung der Gitterstruktur 10 kommt vorzugsweise ein physikalisches Gasphasenabscheideverfahren (PVD - physical vapor deposition), vorzugsweise kombiniert mit einem Ätzverfahren, zum Einsatz. Diese Herstellungsvariante eignet sich besonders zur Herstellung der Verbindungsstege 12, 14, die eine relativ kleine Stegbreite aufweisen. Zumindest bis zu einer Stegbreite der Verbindungsstege 12, 14 von höchstens 25 µm, insbesondere höchstens 20µm, insbesondere höchstens 15 µm, hat sich das kombinierte Verfahren, das sich aus dem PVD-Verfahren, insbesondere einem Sputterverfahren, und einem Ätzverfahren zusammensetzt, als besonders vorteilhaft erwiesen.

### Bezugszeichenliste

- 10: Gitterstruktur
- 11: erster Stabilisierungssteg
- 12: erster Verbindungssteg
- 13: zweiter Stabilisierungssteg
- 14: zweiter Verbindungssteg
- 15: Zelle
- 16, 16', 16", 16"': Stabilisierungsstegreihe
- 21: erster Verbinder
- 22: zweiter Verbinder
- 23: dritter Verbinder
- 24: vierter Verbinder
- 30: Blutgefäß

- P_{L}: Projektion der Längsachse
- P_{Q}: Projektion der Querachse
- b₁: erste Stegbreite
- b₂: zweite Stegbreite
- l₁: erste Steglänge
- l₂: zweite Steglänge

## Patentansprüche

1. Medizinische Vorrichtung mit einer radial komprimierbaren und expandierbaren Gitterstruktur (10), die wenigstens eine geschlossene Zelle (15) aufweist, die durch jeweils vier einstückig miteinander gekoppelte Stege (11, 12, 13, 14) begrenzt ist, von denen zwei Stege (11, 12, 13, 14) als Stabilisierungsstege (11, 13) mit einer ersten Stegbreite b₁ und zwei weitere Stege (11, 12, 13, 14) als Verbindungsstege (12, 14) mit einer zweiten Stegbreite b₂ ausgebildet sind, wobei das Verhältnis b₁/b₂ zwischen der ersten Stegbreite b₁ und der zweiten Stegbreite b₂ mindestens 1,2 beträgt, wobei die Verbindungsstege (12, 14) und die Stabilisierungsstege (11, 13) innerhalb der Zelle (15) jeweils parallel gegenüberliegend angeordnet und die Verbindungsstege (12, 14) durch die Stabilisierungsstege (11, 13) miteinander verbunden sind, und wobei die Verbindungsstege (12, 14) S-förmig ausgebildet sind.

2. Vorrichtung nach Anspruch 1,
**dadurch gekennzeichnet, dass**
das Verhältnis b₁/b₂ zwischen der ersten Stegbreite b₁ und der zweiten Stegbreite b₂ mindestens 1,5, insbesondere mindestens 1,8, insbesondere mindestens 2, insbesondere mindestens 2,5, insbesondere mindestens 3, insbesondere mindestens 4, insbesondere mindestens 5, insbesondere mindestens 6, insbesondere mindestens 8, insbesondere mindestens 10, insbesondere mindestens 12, beträgt.

3. Vorrichtung nach einem der vorhergehenden Ansprüche,
**dadurch gekennzeichnet, dass**
die Gitterstruktur (10) mehrere benachbarte geschlossene Zellen (15) aufweist, deren Stabilisierungsstege (11, 13) miteinander fluchtend verbunden sind derart, dass wenigstens eine Stabilisierungsstegreihe (16) gebildet ist, die sich helixförmig um eine Längsachse der Gitterstruktur (10) windet.

4. Vorrichtung nach Anspruch 3,
**dadurch gekennzeichnet, dass**
wenigstens zwei Stabilisierungsstegreihen (16) gebildet sind, die durch die Verbindungsstege (12, 14), miteinander verbunden sind.

5. Vorrichtung nach Anspruch 4,
**dadurch gekennzeichnet, dass**
die Verbindungsstege (12, 14) in regelmäßigen Abständen zwischen den Stabilisierungsstegreihen (16) angeordnet sind.

6. Vorrichtung nach einem der vorhergehenden Ansprüche,
**dadurch gekennzeichnet, dass**
die Verbindungsstege (12, 14) benachbarter geschlossener Zellen (15) parallel zueinander angeordnet sind.

7. Vorrichtung nach einem der vorhergehenden Ansprüche,
**dadurch gekennzeichnet, dass**
die Stabilisierungsstege (11, 13) im Wesentlichen einen geradlinigen Verlauf aufweisen.

8. Vorrichtung nach einem der vorhergehenden Ansprüche,
**dadurch gekennzeichnet, dass**
die Stabilisierungsstege (11, 13) eine erste Steglänge l₁ und die Verbindungsstege (12, 14) eine zweite Steglänge l₂ aufweisen, wobei die erste Steglänge l₁ und die zweite Steglänge l₂ unterschiedlich sind.

9. Vorrichtung nach Anspruch 8,
**dadurch gekennzeichnet, dass**
das Verhältnis der ersten Steglänge l₁ zur zweiten Steglänge l₂ (l₁/l₂) mindestens 1,2, insbesondere mindestens 1,5, insbesondere mindestens 2, insbesondere mindestens 2,5, insbesondere mindestens 3, insbesondere mindestens 4, insbesondere mindestens 5, beträgt.

10. Vorrichtung nach Anspruch 8,
**dadurch gekennzeichnet, dass**
das Verhältnis der zweiten Steglänge l₂ zur ersten Steglänge l₁ (l₂/l₁) mindestens 1,2, insbesondere mindestens 1,5, insbesondere mindestens 2, insbesondere mindestens 2,5, insbesondere mindestens 3, insbesondere mindestens 4, insbesondere mindestens 5, beträgt.

11. Vorrichtung nach einem der Ansprüche 3 bis 10,
**dadurch gekennzeichnet, dass**
die Gitterstruktur (10) höchstens 8, insbesondere höchstens 6, insbesondere höchstens 4, insbesondere höchstens 3, insbesondere höchstens 2, Stabilisierungsstegreihen (16) aufweist.

12. Vorrichtung nach einem der vorhergehenden Ansprüche
**dadurch gekennzeichnet, dass**
die Gitterstruktur (10) in Umfangsrichtung wenigstens 6, insbesondere wenigstens 8, insbesondere wenigstens 12, insbesondere wenigstens 18, insbesondere wenigstens 24, insbesondere wenigstens 32, insbesondere wenigstens 36, insbesondere wenigstens 48, insbesondere wenigstens 56, insbesondere wenigstens 64, Verbindungsstege (12, 14) aufweist.

13. Vorrichtung nach einem der vorhergehenden Ansprüche,
**dadurch gekennzeichnet, dass**
die Gitterstruktur (10) in Umfangsrichtung höchstens 120, insbesondere höchstens 100, insbesondere höchstens 80, insbesondere höchstens 70, insbesondere höchstens 60, insbesondere höchstens 50, Verbindungsstege (12, 14) aufweist.

14. Vorrichtung nach einem der vorhergehenden Ansprüche,
**dadurch gekennzeichnet, dass**
die zweite Stegbreite b₂ höchstens 35 µm, insbesondere höchstens 30 µm, insbesondere höchstens 25 µm, insbesondere höchstens 20 µm, insbesondere höchstens 15 µm, beträgt.

## Claims

1. A medical device having a radially compressible and expandable lattice structure (10), which comprises at least one closed cell (15), each being delimited by four webs (11, 12, 13, 14) coupled to one another in one piece, of which two webs (11, 12, 13, 14) are configured as stabilisation webs (11, 13) with a first web breadth b₁ and two further webs (11, 12, 13, 14) are configured as connecting webs (12, 14) with a second web breadth b₂, wherein the ratio b₁/b₂ between the first web breadth b₁ and the second web breadth b₂ is at least 1.2, wherein the connecting webs (12, 14) and the stabilisation webs (11, 13) are respectively disposed within the cell (15) such that they lie parallel and opposite to one another and the connecting webs (12, 14) are connected together via the stabilisation webs (11, 13), and wherein the connecting webs (12, 14) are in an S-shaped configuration.

2. The device as claimed in claim 1,
**characterized in that**
the ratio b₁/b₂ between the first web breadth b₁ and the second web breadth b₂ is at least 1.5, in particular at least 1.8, in particular at least 2, in particular at least 2.5, in particular at least 3, in particular at least 4, in particular at least 5, in particular at least 6, in particular at least 8, in particular at least 10, in particular at least 12.

3. The device as claimed in one of the preceding claims,
**characterized in that**
the lattice structure (10) comprises a plurality of adjacent closed cells (15), the stabilisation webs (11, 13) being connected to one another in an aligned manner such that at least one row of stabilisation webs (16) is formed which is helically wound about a longitudinal axis of the lattice structure (10).

4. The device as claimed in claim 3,
**characterized in that**
at least two rows of stabilisation webs (16) are formed, which are connected to one another via the connecting webs (12, 14).

5. The device as claimed in claim 4,
**characterized in that**
the connecting webs (12, 14) are disposed at regular intervals between the rows of stabilisation webs (16).

6. The device as claimed in one of the preceding claims,
**characterized in that**
the connecting webs (12, 14) of adjacent closed cells (15) are disposed parallel to one another.

7. The device as claimed in one of the preceding claims,
**characterized in that**
the stabilisation webs (11, 13) follow an essentially linear course.

8. The device as claimed in one of the preceding claims,
**characterized in that**
the stabilisation webs (11, 13) have a first web length l₁ and the connecting webs (12, 14) have a second web length l₂, wherein the first web length l₁ and the second web length l₂ are different.

9. The device as claimed in claim 8,
**characterized in that**
the ratio of the first web length l₁ to the second web length l₂ (l₁/l₂) is at least 1.2, in particular at least 1.5, in particular at least 2, in particular at least 2.5, in particular at least 3, in particular at least 4, in particular at least 5.

10. The device as claimed in claim 8,
**characterized in that**
the ratio of the second web length l₂ to the first web length l₁ (l₂/l₁) is at least 1.2, in particular at least 1.5, in particular at least 2, in particular at least 2.5, in particular at least 3, in particular at least 4, in particular at least 5.

11. The device as claimed in one of claims 3 to 10,
**characterized in that**
the lattice structure (10) contains at most 8, in particular at most 6, in particular at most 4, in particular at most 3, in particular at most 2 rows of stabilisation webs (16).

12. The device as claimed in one of the preceding claims,
**characterized in that**
in the circumferential direction, the lattice structure (10) comprises at least 6, in particular at least 8, in particular at least 12, in particular at least 18, in particular at least 24, in particular at least 32, in particular at least 36, in particular at least 48, in particular at least 56, in particular at least 64 connecting webs (12, 14).

13. The device as claimed in one of the preceding claims,
**characterized in that**
in the circumferential direction, the lattice structure (10) comprises at most 120, in particular at most 100, in particular at most 80, in particular at most 70, in particular at most 60, in particular at most 50 connecting webs (12, 14).

14. The device as claimed in one of the preceding claims,
**characterized in that**
the second web breadth b₂ is at most 35 µm, in particular at most 30 µm, in particular at most 25 µm, in particular at most 20 µm, in particular at most 15 µm.

## Revendications

1. Dispositif médical comportant une structure grillagée compressible et dilatable radialement (10) qui présente au moins une cellule fermée (15) qui est limitée par quatre traverses (11,12,13,14) respectivement couplées entre elles en formant une seule pièce, dont deux traverses (11,12,13,14) sont réalisées sous forme de traverses de stabilisation (11,13) ayant une première largeur de traverses b1 et deux autres traverses (11,12,13,14) faisant office de traverses de connexion (12,14) ayant une seconde largeur de traverses b2, le rapport b1/b2 entre la première largeur de traverses b1 et la seconde largeur de traverses b2 étant d'au moins 1,2, les traverses de connexion (12,14) et les traverses de stabilisation (11,13) étant disposées respectivement parallèlement opposées dans la cellule (15) et les traverses de connexion (12,14) étant reliées entre elles par les traverses de stabilisation (11,13) et les traverses de connexion (12,14) étant réalisées en forme de S.

2. Dispositif selon la revendication 1,
**caractérisé en ce que**
le rapport b1/b2 entre la première largeur de traverses b1 et la seconde largeur de traverses b2 est d'au moins 1,5, en particulier d'au moins 1,8, en particulier d'au moins 2, en particulier d'au moins 2,5, en particulier d'au moins 3, en particulier d'au moins 4, en particulier d'au moins 5, en particulier d'au moins 6, en particulier d'au moins 8, en particulier d'au moins 10, en particulier d'au moins 12.

3. Dispositif selon une des revendications précédentes,
**caractérisé en ce que**
la structure grillagée (10) présente plusieurs cellules fermées voisines (15) dont les traverses de stabilisation (11,13) sont reliées en alignement mutuel de manière à ce que soit constituée au moins une rangée de traverses de stabilisation (16) qui s'enroule en spirale autour d'un axe longitudinal de la structure grillagée (10).

4. Dispositif selon la revendication 3,
**caractérisé en ce que**
sont constituées au moins deux rangées de traverses de stabilisation (16) qui sont reliées entre elles par les traverses de connexion (12,14).

5. Dispositif selon la revendication 4,
**caractérisé en ce que**
les traverses de connexion (12,14) sont disposées à intervalles réguliers entre les rangées de traverses de stabilisation (16).

6. Dispositif selon une des revendications précédentes,
**caractérisé en ce que**
les traverses de connexion (12,14) de cellules fermées voisines (15) sont disposées parallèlement les unes aux autres.

7. Dispositif selon une des revendications précédentes,
**caractérisé en ce que**
les traverses de stabilisation (11,13) présentent une extension sensiblement rectiligne.

8. Dispositif selon une des revendications précédentes,
**caractérisé en ce que**
les traverses de stabilisation (11,13) présentent une première longueur de traverses l1 et les traverses de connexion (12,14) une seconde longueur de traverses l2, la première longueur de traverses l1 et la seconde longueur de traverses l2 étant différentes.

9. Dispositif selon la revendication 8,
**caractérisé en ce que**
le rapport (11/12) entre la première longueur de traverses l1 et la seconde longueur de traverses l2 est d'au moins 1,2, en particulier d'au moins 1,5, en particulier d'au moins 2, en particulier d'au moins 1,5, en particulier d'au moins 2, en particulier d'au moins 2,5, en particulier d'au moins 3, en particulier d'au moins 4, en particulier d'au moins 5.

10. Dispositif selon la revendication 8,
**caractérisé en ce que**
le rapport (l2/l1) entre la seconde longueur de traverses l2 et la première longueurs de traverses l1 est d'au moins 1,2, en particulier d'au moins 1,5, en particulier d'au moins 2, en particulier d'au moins 2,5, en particulier d'au moins 3, en particulier d'au moins 4, en particulier d'au moins 5.

11. Dispositif selon une des revendications 3 à 10,
**caractérisé en ce que**
la structure grillagée (10) présente au plus 8, en particulier au plus 6, en particulier au plus 4, en particulier au plus 3, en particulier au plus 2 rangées de traverses de stabilisation (16).

12. Dispositif selon une des revendications précédentes,
**caractérisé en ce que**
la structure grillagée (10) présente dans le sens circonférentiel au moins 6, en particulier au moins 12, en particulier au moins 18, en particulier au moins 24, en particulier au moins 32, en particulier au moins 36, en particulier au moins 48, en particulier au moins 56, en particulier au moins 64 traverses de connexion (12,14).

13. Dispositif selon une des revendications précédentes,
**caractérisé en ce que**
la structure grillagée (10) présente dans le sens circonférentiel au plus 120, en particulier au plus 80, en particulier au plus 70, en particulier au plus 60, en particulier au plus 50 traverses de connexion (12,14).

14. Dispositif selon une des revendications précédentes,
**caractérisé en ce que**
la seconde largeur de traverses b2 est d'au plus 35 µm, en particulier d'au plus 30 µm, en particulier d'au plus 25 µm, en particulier d'au plus 20 µm, en particulier d'au plus 15 µm.
